(19) Europäisches Patentamt
European Patent Office
Office européen des brevets

(11) Publication number: **0 237 472**
**A2**

(12) # EUROPEAN PATENT APPLICATION

(21) Application number: 87810064.3

(51) Int.Cl.[3]: **A 61 K 31/385**

(22) Date of filing: 30.01.87

(30) Priority: 05.02.86 GB 8602841

(43) Date of publication of application:
16.09.87 Bulletin 87/38

(84) Designated Contracting States:
BE CH DE FR GB IT LI LU NL SE

(71) Applicant: Zyma SA
Route de l'Etraz
CH-1260 Nyon(CH)

(71) Applicant: NIHON NOHYAKU CO., LTD.
1-2-5, Nihonbashi
Chuo-ku Tokyo(JP)

(72) Inventor: Dumont, Jean-Maurice
chemin des Morettes 7A
CH-1197 Prangins/Vaud(CH)

(72) Inventor: Ryhänen, Lasse Johannes, Dr.
Luokotie 1 as 7
SF-90530 Oulu 53(FI)

(74) Representative: Meyer, Hans Rudolf et al,
Patentabteilung der CIBA-GEIGY AG Postfach
CH-4002 Basel(CH)

(54) **An ameliorating agent for pulmonary fibrosis and the use thereof.**

(57) Compounds of the formula (I),

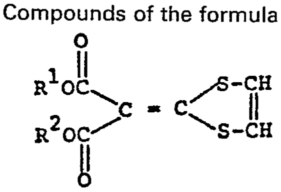

(I)

wherein $R^1$ and $R^2$ are the same or different and represent alkyl groups of $C_1$ to $C_4$ are useful as ameliorating agents for pulmonary fibrosis.

EP 0 237 472 A2

# An ameliorating agent for pulmonary fibrosis and the use thereof

The present invention is related to an ameliorating agent for pulmonary fibrosis which comprises a compound represented by the general formula (I),

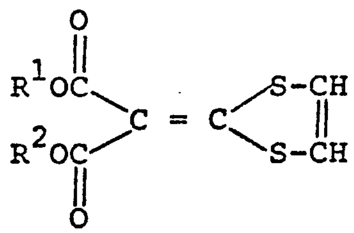

(I)

wherein $R^1$ and $R^2$ are the same or different and represent alkyl groups of $C_1$ to $C_4$, and a pharmaceutically acceptable diluent; and the use thereof.

$C_1$-$C_4$-alkyl is especially methyl, ethyl, n-propyl, isopropyl, n-butyl or isobutyl; but may be also sec-butyl or tert -butyl.

The dithiol derivatives represented by the general formula (I) (hereinafter referred to as the present compounds) are known ones as a component for pharmaceutical compositions for controlling the liver diseases (cp. e.g. GB-A-1,509,819 or GB-A-1,513,936). The present inventors have completed the present invention based on the findings, as a result of intensive researches on the pharmacological effects of these compounds represented by the general formula (I), that the present compounds have an unexpected pharmacological effect which can not be anticipated from the previous findings. That is, we have found that the present compounds can ameliorate pulmonary fibrosis, when given in vivo.

In other words, the present invention can not be conceived from the previous findings, the controlling of the liver diseases; since the cause of the pulmonary fibrosis is considered to be entirely different from that of the liver

diseases. The function of the lungs, of course, is very important for the maintenance of the life. If the collagen which is a necessary connective tissue for preserving the normal construction of the lung and the function thereof increases abnormally to form diffuse fibroplasia, this leads to its functional disorder. This is a typical pulmonary fibrosis, however, there are neither effective therapeutical means nor ameliorating methods while the normalization and/or ameriolation of such situation is very important. The pulmonary fibrosis is caused by bronchitis, collagenosis, dusts such as silica particles, certain medicines and x-ray radiation. There are pathological and biochemical methods for the diagnostics for the present disease. The latter method is practical and in this method usually the level of hydroxyproline which is an index for collagen is assayed (Hesterberg, T.W., Bleomycin-induced pulmonary fibrosis: Correlation of biochemical, physiological and histological change Tox. & Appl. Pharmacol. 60, 360-367 (1981)).

In order to study the pulmonary fibrosis, the experimental works are very important as well as clinical ones.

The pulmonary fibrosis can be induced by bleomycin, silica particles, ozone or $CCl_4$ (Adamson, I.Y.R., Bowden, D.H.: The pathogenesis of bleomycin-induced pulmonary fibrosis in mice. Am. J. Pathol. 77, 185-190 (1974), Reisen, K.M., Last, J.A.: Silicosis and fibrogenosis: fact and artifact. Toxicology, 13, 51-72 (1979), Hesterberg, T.W., Last, J.D.: Ozone-induced acute pulmonary fibrosis in rats. Am. Rev. Respir. Dis. 123, 47-52 (1981), Anttinen, H., Oikarinen, A., Puistola, U., Paakko, P., Ryhanen, L: Prevention by zinc of rat lung collagen accumulation in carbon tetrachloride injury. Am. Rev. Respir. Dis.132,536-540 (1985).

Futhermore, investigations have also been carried out in order to find out a method for ameliorating the pulmonary

fibrosis by using those experimentally induced fibrosis models (Anttinen, H., et al., ibidem, Pepin, J.M., Effect of dimethyl sulfoxide (DMSO) on bleomycin-induced pumonary fibrosis, Biochemical. Pharmacol. 34, No. 13, 2386-2389, (1985)).

The present inventors have found that the pulmonary fibrosis induced by those agents experimentally to animals can be ameliorated by administering the present compounds.

Therefore, the present compounds are effective as an ameliorating agent for pulmonary fibrosis.

Preferred compounds of the dithiol derivatives represented by the general formula (I) are given in Table 1.

Table 1

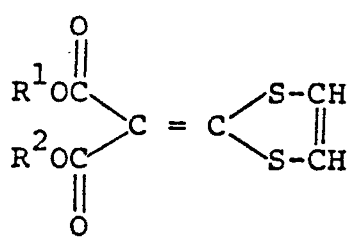

| Compound No. | $R^1$ | $R^2$ | m.p. or refractive index |
|---|---|---|---|
| 1 | $CH_3$ | $CH_3$ | m.p. 134.5° - 135°C |
| 2 | $C_2H_5$ | $C_2H_5$ | m.p. 113°C |
| 3 | $n\text{-}C_3H_7$ | $n\text{-}C_3H_7$ | m.p. 73° - 75°C |
| 4 | $i\text{-}C_3H_7$ | $i\text{-}C_3H_7$ | m.p. 59° - 60°C |
| 5 | $n\text{-}C_4H_9$ | $n\text{-}C_4H_9$ | m.p. 55° - 57°C |
| 6 | $i\text{-}C_4H_9$ | $i\text{-}C_4H_9$ | m.p. 76° - 78°C |
| 7 | $C_2H_5$ | $i\text{-}C_3H_7$ | m.p. 57° - 58°C |
| 8 | $CH_3$ | $i\text{-}C_4H_9$ | $n_D^{20}$ 1.5928 |

The number of the compound used here in the present specification denotes the one which corresponds to the number in Table 1 shown above.

The present compounds are extremely low toxic to mammals, and the $LD_{50}$ values of the acute oral toxicities to male mice are such lower level as in the range from 1,000 to 6,000 mg/kg of body weight or more, in general. The toxicities of the preferred compounds given in Table 1 are as follows:

| Compound no. | LD50 Values |
|---|---|
| 1 | >6,000 |
| 2 | 4,900 |
| 3 | >6,000 |
| 4 | 3,120 |
| 5 | >6,000 |
| 6 | 4,700 |
| 7 | 2,700 |
| 8 | 5,800 |

Further, these compounds have no detrimental effects on test animals administered therewith, so far as the doses thereof are within an ordinary administration range.

In using the compounds represented by general formula (I) according to the present invention, they are used in the form suitable for exhibiting the intended efficacy as an ameliorating agent for pulmonary fibrosis. They may also be formulated, according to usual procedures and means adopted in this field, into pharmaceutical compositions in the form of a mixture with a pharmaceutically acceptable diluent and/or other pharmacologically active substance. Otherwise, it is also permitted to provide the pharmaceutical compositions of this invention in the form of administration units convenient for their individual application purposes

which contain the compound of this invention alone or contain it in mixture with a pharmaceutically acceptable diluent and/or other pharmacologically active substance.

The pharmaceutical compositions of this invention can be administered either orally or parenterally. Accordingly, the pharmaceutical of this invention can take an appropriate shape suitable for oral or parenteral administration.

It is also permitted to provide the pharmaceutical of this invention in the form of administration units. In this case, the pharmaceutical composition contains an effective amount of active ingredient compound. Said effective amount should mean a certain range in accordance with the changes in various conditions.

The shape in which the pharmaceutical compositions of this invention can be provided includes, for example, powders, granules, tablets, sugar-coated tablets, pills, capsules, suppositories, suspensions, liquids, emulsions, ampoules, injections, inhalants and the like.

The pharmaceutical compositions of this invention are provided, in accordance with needs, in the aseptic state or in the form of an isotonic aqueous solution.

This invention includes such mode that the compound of general formula (I) is administered in the form of a mixture with a pharmaceutically acceptable diluent. The diluent referred to herein means a material other than the compounds represented by general formula (I). The diluent may be a solid, a semisolid, a liquid or an ingestible capsule. Examples of the diluent include vehicles, extenders, binders, wetting agents, disintegrators, surfactants, lubricants, dispersants, buffer agents, seasonings, deodorants, dyes, flavors, preservatives, dissolution aids,

solvents, propellants and the like, though these are not limitative. These diluents may be used either singly or in the form of a mixture of two or more members. Such pharmaceutically acceptable diluents as above are sometimes used in the form of a mixture with other pharmacologically active substance, which also belongs to the scope of this invention.

The pharmaceutical composition of this invention may be prepared according to any known method. For example, a mixture of the active ingredient and a diluent is formed, for example, into granules, and thus formed granular composition is molded, for example, into tablets. In case the pharmaceutical composition is for parenteral administration, it is preferable to be made aseptic and, if necessary, be made isotonic to the blood.

Generally, the pharmaceutical composition of this invention contains about 0.01 to 100% by weight, based on the weight of the composition, of the active compound. Thus, this invention includes such mode that the said compound is used independently.

In case the compound of this invention is formulated into administration units, individual constituents forming the formulated composition may be either same or different in the shape. Most frequently adopted shapes include, for example, the followings: tablets, granules, pills, powders, sugar-coated tablets, capsules, ampoules and the like.

For the amelioration of the pulmonary fibrosis, the pharmaceutical composition of the present invention may be applied to mammals including humans according to an ordinary procedure adopted in this field, in order to attain such effects as shown in the aforesaid animal tests. Thus, the composition of the present invention is administered orally

or parenterally. The oral administration includes sublingual administration and inhalation, and the parenteral administration includes administration by way of injection including, for example, subcutaneous, intramuscular and intravenous injections and instillation.

The dose of the pharmaceutical composition of this invention varies depending on many factors, including the kind of mammal. the difference in susceptibility, age, sex, body weight, the clinical picture, the physical conditions of patients, the means of administration, the time and interval of administration, the kind and properties of pharmaceutical composition, the kind of active ingredient, etc. In some cases, accordingly, the dose of the pharmaceutical composition may be made smaller than the minimum dose mentioned below, while in other cases the dose would be in excess of the maximum dose mentioned below. In case the pharmaceutical composition is to be administered in a large dose, it is preferable that the pharmaceutical composition is divisionally administered several times a day.

In the case of oral administration, effective dose for animals is in the range from 0.1 to 500 mg, preferably from 1 to 100 mg, of active ingredient per one kilogramme body weight per day. In the case of parenteral administration, effective dose for animals is in the range from 0.01 to 250 mg, preferably from 0.1 to 25 mg, of active ingredient per one kilogramme body weight per day.

In the case of oral administration, effective dose for humans , deduced from the above-mentioned effective dose for animals with consideration for susceptibility difference and security, is advantageously in the range from 0.1 to 250 mg, preferably from 0.5 to 50 mg, per one kilogramme body weight

per day. In the case of parenteral administration, effective dose for humans is in the range from 0.01 to 100 mg, preferably from 0.1 to 25 mg, per one kilogramme body weight per day.

The present invention is illustrated in more detail below with reference to examples wherein all parts are by weight. The kinds and ratios of the components to be employed may be modified as needed.

Example 1

| | |
|---|---|
| Compound 2 | 10 parts |
| Heavy magnesium oxide | 10 " |
| Lactose | 80 " |

The above-mentioned components were homogeneously mixed to obtain a powder or granule.

Example 2

| | |
|---|---|
| Compound 3 | 10 parts |
| Synthetic aluminum silicate | 10 " |
| Calcium hydrogenphosphate | 5 " |
| Lactose | 75 " |

The above-mentioned components were treated in the same manner as in Example 1 to obtain a powder.

Example 3

| | |
|---|---|
| Compound 4 | 50 parts |
| Starch | 10 " |
| Lactose | 15 " |
| Crystalline cellulose | 20 " |
| Plyvinyl alcohol | 5 " |
| Water | 30 " |

The above-mentioned components were homogeneously kneaded, granulated, dried and sieved to obtain a granule.

Example 4

99 Parts of the granule obtained in Example 3 was incorporated with 1 part of calcium stearate, and then subjected to compression molding to obtain a tablet of 10 mm in diameter.

Example 5

| | |
|---|---|
| Compound 7 | 95 parts |
| Polyvinyl alcohol | 5 " |
| Water | 30 " |

The above-mentioned components were treated in the same manner as in Example 3 to obtain a granule. 90 parts of the thus obtained granule was incorporated with 10 parts of crystalline cellulose, and then subjected to compression molding to obtain a tablet of 8 mm in diameter. Further, this tablet was formed into a sugar-coated tablet by use of proper amounts of a suspention comprising syrup, gelatin and precipitated calcium carbonate and a pigment.

Example 6

| | |
|---|---|
| Compound 4 | 0.5 part |
| Nonionic surfactant | 2.5 parts |
| Isotonic sodium chloride solution | 97.0 " |

The above-mentioned components were mixed together with heating to form a solution, which was then cooled, and sterilized to obtain an injection.

Example 7

The powder obtained in Example 1 was filled into commercially available capsules to obtain a capsule.

The experimental examples are given below.

Experimental example 1

METHODS

Animals

The animals used were female Sprague-Dawley rats fed on a commercial diet (Hankkija Cy, Helsinki, Finland). Forty rats were divided into four groups of 10, the first serving as a control group. In order to investigate the effect of Compound 4 on lung collagen accumulation, the injury was induced by injecting carbon tetrachloride ($CCl_4$) intraperitoneally at doses of 0.1 ml/100 g body weight (diluted with an equal volume of paraffin oil) twice a week for 40 days. The third group received simultaneous doses of $CCl_4$ and Compound 4. Compound 4 was suspended in arabic gum and given by gastric intubation at doses of 100 mg/kg body

weight 5 times weekly for 40 days. The fourth group received only Compound 4. Two animals both in the $CCl_4$ group died during the experiment.

Assay Procedures

At the end of the experiment, the rats were anaesthetized with ether, weighed, and decapitated. The lungs were rapidly removed, weighed and the right one of them stored at -70°C until assayed. Tissue specimens for the determination of hydroxyproline and total protein were homogenized with an Ultra-Turrax homogenizer at 0°C in 0.5 M acetic acid (5 ml/g of tissue), dialyzed against 0.5 M acetic acid, and hydrolyzed in 6 M HCl at 120°C overnight. Tissue hydroxyproline was assayed by the method of Kivirikko et al. and tissue total protein using the α-amino nitrogen method of Rubinstein and Pryce.

Inflation-fixation Procedure

The left lung was fixed by slowly injecting a 4% formaldehyde and 1% glutaraldehyde solution into the main bronchus with the 1 ml syringe under magnifying lens control. Inflation of the lungs with fixative was continued until the pleura was smooth and the organ appeared to be filled. The bronchus was then left unclosed and the lungs were placed in the same solution until fixed. When the bronchus is left unclosed, the human lungs, at least, seem to assume a fairly constant volume during fixation, a little greater than the functional residual capacity. The lungs were then sliced sagittally and the slices embedded in paraffin. Sections were cut at 5 μm and stained with heamatoxylin-eosin, Pearl's iron, van Gieson's and Verhoeff's elastic stains.

## Results and Discussion

### Induction of Lung Fibrosis by $CCl_4$: the Effect of the present compound

The total dose of 1.2 ml of $CCl_4$ per 100 g of body weight received by the rats significantly increased the lung weight to body weight ratio and lung total protein content, while the relative protein content of the lungs decreased. Compound 4, given orally, was able to normalize these parameters completely (see Table 2). $CCl_4$ induction also caused a significant increase in the total hydroxyproline content of the lungs and here again Compound 4 treatment normalized the values (see Figure 1).

### Effect of the Present Compound on the Morphology of $CCl_4$ Injury

The alveolar pattern in the lungs of the ten control rats and the ten which received Compound 4 was normal, but in eight out of the nine lungs from rats with $CCl_4$ induction the alveolar walls were widened almost throughout the whole organ and infiltrated with inflammatory cells, including mostly macrophages and lymphocytes and only occasional neutrophils. Some airspaces contained abundant alveolar oedema and macrophages, which often showed cytoplasmic vacuolisation. All the lungs exhibited foci of necrotic alveolar walls. Five out of the nine lungs from rats which had received $CCl_4$ and Compound 4 treated rats exhibited a fairly normal alveolar pattern, while four showed variable evidence of inflammatory reaction with minimal oedema. These lungs also showed less interstitial fibrosis than those from the $CCl_4$-induced rats which had not received Compound 4. All the lungs showed some foci of necrotic alveolar walls, however, indicating diffuse alveolar damage.

Table 2

Effect of $CCl_4$ and Compound 4 on Lung to Total Body Weight Ratio and Lung Protein Content.

| Group | | Lung weight/ body weight (%) | Lung total protein (mg/lungs) | Lung relative protein (mg/g of lung weight) |
|---|---|---|---|---|
| Control | (C) | 0.86±0.19 | 40.88±6.38 | 22.79±2.89 |
| Compound 4 | (M) | 0.81±0.12 | 38.31±4.90 | 22.20±2.76 |
| $CCl_4$ | (I) | 1.49±0.31* | 48.14±8.91** | 16.40±2.14*** |
| $CCl_4$ + Compound 4 | (M+I) | 0.89±0.17 | 40.07±6.05 | 20.61±2.05 |

Significant differences from all other groups in one-way analyses of variance

* : P<0.1, ** : P<0.05, *** : P<0.01

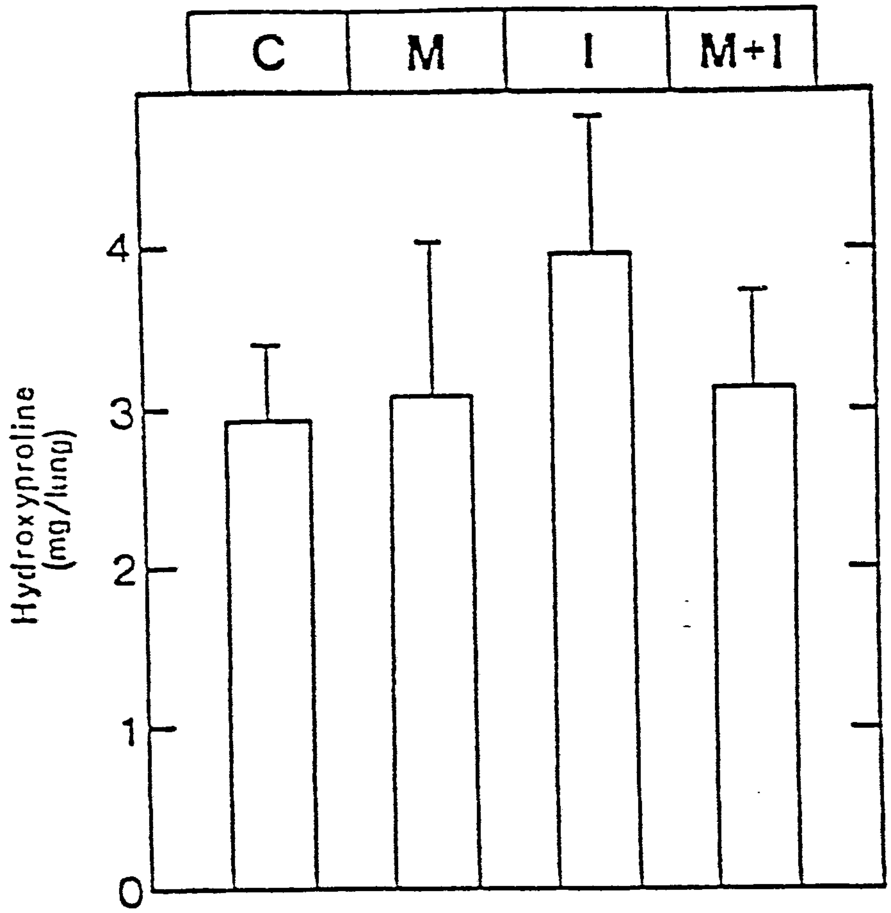


Figure 1. Effect of Compound 4 on Lung Hydroxyproline Content in $CCl_4$-induced Damages.

Treatment with Compound 4 completely normalized the lung weight to body weight ratio, lung total and relative protein content and total hydroxyproline content. Morphologically, Compound 4 seemed to prevent the exudative inflammatory changes and interstitial fibrosis, but not the primary toxic effect of $CCl_4$ on the capillary endothelium or the alveolar epithelium, the result of which was diffuse alveolar damage. It is concluded that Compound 4 can be a useful drug for the prevention of pulmonary fibrosis.

4. Brief Explanation of the Drawings

Figure 1 shows the effect of Compound 4 on lung hydroxyproline content in $CCl_4$-induced damages wherein C denotes control group, M denotes Compound 4 group, I denotes $CCl_4$-group and M + I denotes $CCl_4$ group and Compound 4.

Claims

1. An ameliorating agent for pulmonary fibrosis which comprises a dithiol derivative represented by the general formula (I),

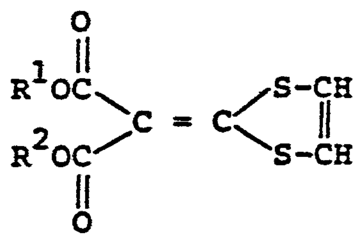

(I)

wherein $R^1$ and $R^2$ are the same or different and represent alkyl groups of $C_1$ to $C_4$, and a pharmaceutically acceptable diluent.

2. An ameliorating agent for pulmonary fibrosis according to Claim 1, wherein said dithiol derivative is diisopropyl 1,3-dithiol-2-ylidene malonate.

3. The use of a compound represented by the general formula (I),

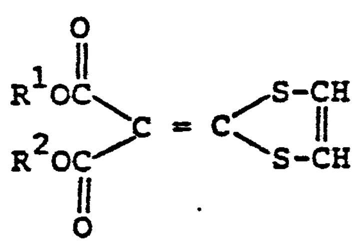

(I)

wherein $R^1$ and $R^2$ are the same or different and represent alkyl groups of $C_1$ to $C_4$, for the manufacture of a medicament for the ameliorative treatment of pulmonary fibrosis.

4. The use as recited in Claim 3, wherein said compound is diisopropyl 1,3-dithiol-2-ylidene malonate.

5. The use of a compound of formula I defined in Claim 3 for the amelioration of pulmonary fibrosis.

6. The use as recited in Claim 5, wherein said compound is diisopropyl 1,3-dithiol-2-ylidene malonate.

7. A method of ameliorating pulmonary fibrosis comprising the the administration to a mammal in need thereof of a therapeutically effective amount of a compound of formula I defined in Claim 3.

8. A method according to Claim 7 comprising the administration of a therapeutically effective amount of diisopropyl 1,3-dithiol-2-ylidene malonate.